# EUROPÄISCHE PATENTANMELDUNG

(11) **EP 4 745 401 A1**
(43) Veröffentlichungstag der Anmeldung: **20.05.2026**
(21) Anmeldenummer: 24213021.9
(22) Anmeldetag: 14.11.2024
(51) Int. Cl.: F04B 43/12, F04B 53/22

(54) **MEDIZINISCHES SCHLAUCHPUMPEN-AGGREGAT**

(71) Anmelder: Richard Wolf GmbH, 75438 Knittlingen (DE)
(72) Erfinder: Steinhauser, Luis, 75438 Knittlingen (DE); Tewes, Moritz, 75438 Knittlingen (DE); Knoth, Stefan, 75438 Knittlingen (DE); Lampert, Alexander, 75248 Ölbronn-Dürrn (DE)
(74) Vertreter: Patentanwälte Hemmer Lindfeld Frese Partnerschaft mbB

(57) **Zusammenfassung**

Die Erfindung betrifft ein medizinisches Schlauchpumpen-Aggregat mit einem Pumprad (6) und zumindest einem das Pumprad (6) in einem Umfangsbereich umschließenden Pumpschlauch (30), wobei der Pumpschlauch (30) in seinem das Pumprad (6) umschließenden Abschnitt an seiner dem Pumprad (6) abgewandten Außenseite an einem Widerlager (40) anliegt, sowie eine zugehörige Schlauchkassette.

## Beschreibung

Die Erfindung betrifft ein medizinisches Schlauchpumpen-Aggregat sowie eine Schlauchkassette für ein solches medizinisches Schlauchpumpen-Aggregat.

Medizinische Schlauchpumpen-Aggregate werden beispielsweise in Verbindung mit endoskopischen Operationssystemen zum Absaugen von Flüssigkeit aus dem Operationsgebiet verwendet. Bei derartigen Perestaltikpumpen ist ein Pumpschlauch um ein Pumprad geführt, welches an seinem außen Umfang Verdrängerelemente aufweist, welche bei Rotation des Pumprades den Pumpschlauch umlaufend komprimieren und entspannen, wodurch eine Pump- bzw. Förderbewegung im Inneren des Pumpschlauches verursacht wird. Um eine ausreichende Pumpwirkung zu erzielen, gleichzeitig aber gewünschte Standzeiten einzuhalten bzw. Verschleiß zu minimieren, ist es wichtig, die Spannung des Pumpschlauches am Außenumfang des Pumprades richtig einzustellen.

Es ist Aufgabe der Erfindung ein medizinisches Schlauchpumpenaggregat bereitzustellen, welches auf einfache Weise eine korrekte Positionierung des Pumpschlauches am Pumprad sicherstellt.

Diese Aufgabe wird gelöst durch ein medizinisches Schlauchpumpen-Aggregat mit den in Anspruch 1 angegebenen Merkmalen sowie durch eine Schlauchkassette für ein medizinisches Schlauchpumpen-Aggregat mit den in Anspruch 12 angegebenen Merkmalen. Bevorzugte Ausführungsformen ergeben sich aus den Unteransprüchen, der nachfolgenden Beschreibung sowie den beigefügten Figuren.

Das medizinische Schlauchpumpen-Aggregat gemäß der Erfindung weist ein Pumprad und zumindest einen das Pumprad in einem Umfangsbereich umgreifenden bzw. umschließenden Pumpschlauch auf. In dem Bereich, in dem der Pumpschlauch das Pumprad umschließt, insbesondere schlingenartig umschließt, ist erfindungsgemäß ein Widerlager vorgesehen. Das Widerlager ist beabstandet zu dem Pumprad so angeordnet, dass der Pumpschlauch mit seiner dem Pumprad entgegengesetzten Seite an dem Widerlager anliegt. So ist der Pumpschlauch in einem Abschnitt zwischen dem Pumprad und dem Widerlager angeordnet. Diese Anordnung hat den Vorteil, dass der Pumpschlauch bei Rotation des Pumprades gegen das Widerlager gedrückt wird, wodurch eine definierte Anlagefläche geschaffen ist, gegen welche der Pumpschlauch zu seinem Verschluss gepresst wird. Dies ermöglicht es, die auf den Pumpschlauch wirkende Spannung gering zu halten. Insbesondere kann im Wesentlichen unabhängig von der Spannung des Pumpschlauches ein ausreichender Verschluss des Pumpschlauches zum Erzielen der Pumpwirkung bzw. eines Unterdrucks im Inneren des Pumpschlauches sichergestellt werden, indem ein Verdrängerkörper des Pumprades den Pumpschlauch fortlaufend gegen das Widerlager drückt.

Das Widerlager wird vorzugsweise von einer Anlagefläche, insbesondere einer bogenförmigen, sich konzentrisch zur Drehachse des Pumprades erstreckenden Anlagefläche gebildet. So erstreckt sich die Anlagefläche vorzugsweise in einem Umfangsbereich des Pumprades parallel zum Außenumfang des Pumprades, d.h. in einem definierten radialen Abstand zur Drehachse. Der Abstand ist vorzugsweise auf den Durchmesser des Schlauches so abgestimmt, dass der Schlauch durch einen Verdrängerkörper gegen die Anlagefläche gedrückt werden kann und dabei verschlossen wird.

Gemäß einer besonderen Ausführungsform der Erfindung kann der zumindest eine Pumpschlauch in einer Schlauchkassette fixiert sein, in welcher auch das Widerlager ausgebildet ist. Der zumindest eine Pumpschlauch kann in der Schlauchkassette beispielsweise formschlüssig, kraftschlüssig und/oder Stoffschlüssig fixiert sein. So kann der Pumpschlauch in der Schlauchkassette definiert relativ zu dem Widerlager angeordnet sein. So kann eine vorgefertigte Baueinheit aus Pumpschlauch und Widerlager geschaffen werden, welche austauschbar in das Schlauchpumpen-Aggregat eingesetzt werden kann.

Gemäß einer weiteren möglichen Ausgestaltung der Erfindung ist das Pumprad an oder in einem Motorgehäuse angeordnet und die Schlauchkassette ist so ausgebildet, dass sie lösbar mit dem Motorgehäuse verbindbar oder in das Motorgehäuse einsetzbar ist, wobei das Pumprad in die Schlauchkassette eingreift und mit dem zumindest einen Pumpschlauch in Kontakt tritt. Wenn das Widerlager in der Schlauchkassette ausgebildet ist, kommt so der Pumpschlauch zwischen dem Pumprad und dem Widerlager in einem Umfangsbereich des Pumprades zu liegen. In diesem Umfangsbereich kann das Pumprad, beispielsweise über einen Verdrängerkörper, auf den Pumpschlauch einwirken, sodass dieser fortlaufend durch Andrücken an das Widerlager verschlossen wird, wodurch ein Flüssigkeitsvolumen in dem Pumpschlauch bewegt bzw. gefördert werden kann.

Der zumindest eine Pumpschlauch ist vorzugsweise mit seinen axialen Enden in der Schlauchkassette fixiert, vorzugsweise jeweils an einem Anschlusselement fixiert. Die Anschlusselemente können in der Schlauchkassette fest angeordnet bzw. einstückig mit weiteren Teilen der Schlauchkassette ausgebildet sein. Die Anschlusselemente sind vorzugsweise jeweils mit einem Anschlussschlauch verbunden oder weisen eine Verbindung für einen solchen Anschlussschlauch auf. So kann der zumindest eine Pumpschlauch getrennt von den Anschlussschläuchen ausgebildet werden und insbesondere in der Schlauchkassette so vormontiert werden, dass eine definierte Positionierung zwischen Pumpschlauch und Widerlager und den Anschlusselementen erreicht wird. Damit kann durch Positionierung der Schlauchkassette auch der zumindest eine Pumpschlauch in eine gewünschte vordefinierte Position relativ zu dem Pumprad gebracht werden. Dies ermöglicht eine sehr einfache Inbetriebnahme des Schlauchpumpen-Aggregats.

Die Schlauchkassette weist weiter bevorzugt eine offene Seitenfläche auf, durch welche das Pumprad in das Innere der Schlauchkassette eingreift bzw. sich in das Innere der Schlauchkassette hinein erstrecken kann. Eine solche Schlauchkassette kann insbesondere dazu ausgebildet sein, an einer Außenseite an das Gehäuse bzw. Motorgehäuse des Schlauchpumpen-Aggregates angesetzt zu werden. Bei einer solchen Ausgestaltung ist das Pumprad bevorzugt außerhalb des Gehäuses an einer Seitenfläche bzw. Stirnfläche des Gehäuses angeordnet und die Schlauchkassette kann an die Außenseite bzw. Außenwandung des Gehäuses bzw. Motorgehäuses so angesetzt werden, dass sie das Pumprad übergreift bzw. das Pumprad in die offene Seitenfläche eingreift und dort mit dem zumindest einen Pumpschlauch in der Schlauchkassette in Eingriff und Wechselwirkung tritt.

Gemäß einer weiteren bevorzugten Ausführungsform ist eine Stirnseite der Schlauchkassette derart offen ausgebildet, dass die Schlauchkassette mit der offenen Stirnseite voran über das Pumprad aufschiebbar ist, wobei vorzugsweise eine das Widerlager bildende Innenwandung dieser offenen Stirnseite zugewandt ist. So kann das Pumprad von der offenen Stirnseite her in die Schlauchkassette eingeführt und auf die das Widerlager bildende Innenwandung zubewegt werden, bis das Pumprad an dem an diesem Widerlager bzw. dieser Innenwandung gelegenen Pumpschlauch zur Anlage kommt, so dass der Pumpschlauch zwischen dem Pumprad und dem Widerlager gelegen ist. Das Pumprad erstreckt sich bei dieser Ausgestaltung vorzugsweise, wie vorangehend beschrieben, durch eine offene Seitenfläche vorzugsweise eine Seitenfläche, welche an die offene Stirnseite angrenzt, in die Schlauchkassette hinein. Dabei sind die offene Stirnseite und die offene Seitenfläche bevorzugt gewinkelt, insbesondere im Wesentlichen um 90° gewinkelt zueinander gerichtet.

Das Pumprad weist vorzugsweise zumindest einen Verdrängerkörper auf, welcher bei Drehung des Pumprades auf den zumindest einen Pumpschlauch einwirkt, wobei der Verdrängerkörper vorzugsweise eine in dem Pumprad drehbar gelagerte Verdrängerrolle ist, welche derart angeordnet ist, dass sie bei der Drehung des Pumprades auf dem Pumpschlauch abrollt. Dabei ist die Verdrängerrolle vorzugsweise so angeordnet, dass sie in dem Bereich, in dem sie mit dem Pumpschlauch in Kontakt ist, den Pumpschlauch gegen das Widerlager zusammendrückt und verschließt. Durch Abrollen der Verdrängerrolle wird somit im Umfangsbereich des Pumprades eine im Pumpschlauch wandernde Verschlussstelle geschaffen. Besonders bevorzug sind an dem Pumprad zwei oder mehr solche Verdrängerkörper, vorzugsweise Verdrängerrollen, über den Umfang gleichmäßig verteilt angeordnet.

Gemäß einer weiteren bevorzugten Ausführungsform kann derzumindest eine Verdrängerkörper, vorzugsweise die zumindest eine Verdrängerrolle in dem Pumprad in radialer Richtung, d.h. in radialer Richtung bezogen auf die Drehachse des Pumprades, federnd gelagert sein. Durch die federnd gelagerten bzw. gefederten Verdrängerkörper kann ein Toleranzausgleich geschaffen werden, sodass auch bei auftretenden Toleranzen stets eine ausreichende Pumpwirkung bzw. ein ausreichender Unterdruck sichergestellt werden kann. Gleichzeitig kann die Kraft zum Einsetzen des Pumpschlauches gering gehalten werden, da keine große Dehnung des Pumpschlauches erforderlich ist.

Gemäß einer speziellen Ausführungsform der Erfindung sind zumindest zwei zueinander parallel verlaufende Pumpschläuche vorgesehen, welche das Pumprad in einem Abschnitt dessen Außenumfangs umschließen. An dem Pumprad sind gleichzeitig vorzugsweise zumindest ein erster Verdrängerkörper, welcher auf einen ersten der Pumpschläuche wirkt, und zumindest ein zweiter Verdrängerkörper, welcher auf den zweiten der Pumpschläuche wirkt, angeordnet. Es können auch mehrere erste und mehrere zweite Verdrängerkörper angeordnet sein, welche jeweils auf den ersten oder den zweiten Pumpschlauch wirken. Die Verdrängerkörper können auch hier bevorzugt Verdrängerrollen sein, die ebenfalls federnd gelagert sein können. Durch die Anordnung von zwei oder mehr Pumpschläuchen kann der Durchfluss durch das Pumpenaggregat erhöht werden. Wenn ferner der zumindest eine erste Verdrängerkörper und der zumindest eine zweite Verdrängerkörper in Umfangsrichtung versetzt an dem Pumprad angeordnet sind, kann darüber hinaus die Pulsation des Schlauchpumpen-Aggregates verringert werden, da in einer Saugphase des ersten Pumpschlauches eine Druckphase des zweiten Pumpschlauches gelegen sein kann und umgekehrt. Eine entsprechende Anordnung von Verdrängerkörper könnte auch bei drei oder mehr zueinander parallel verlaufenden Pumpschläuchen vorgesehen sein.

Die beschriebene Schlauchkassette mit dem zumindest einen Pumpschlauch, weiter bevorzugt den zumindest zwei Pumpschläuchen kann gemäß einer weiteren möglichen Ausgestaltung als austauschbarer, zum Einmalgebrauch vorgesehener Artikel ausgebildet sein. Dies ermöglicht es, den Pumpschlauch ggf. auch mit den Anschlussschläuchen in bzw. an der Schlauchkassette vorzumontieren, sodass diese vormontiere Einheit sehr einfach auszutauschen ist und damit kein großer Reinigungsaufwand gegeben ist, da alle Teile, welche mit der zu fördernden Flüssigkeit in Kontakt kommen, nach der Benutzung entsorgt werden können.

Neben dem vorangehend beschriebenen medizinischen Schlauchpumpen-Aggregat ist Gegenstand der Erfindung eine Schlauchkassette für medizinisches Schlauchpumpen-Aggregat, vorzugsweise für ein solches medizinisches Schlauchpumpen-Aggregat, wie es vorangehend beschrieben wurde. Die Schlauchkassette weist einen Aufnahmeraum für ein Pumprad auf, welcher an einer Seite von einer Anlagefläche begrenzt ist, an welcher ein Pumpschlauch anliegt. Die Anlagefläche bildet dabei ein Widerlager für zumindest ein Verdrängerelement bzw. zumindest einen Verdrängerkörper in einem Schlauchpumpen-Aggregat. Der Pumpschlauch ist bevorzugt in der Schlauchkassette fixiert, beispielsweise mit seinen Enden an in der Schlauchkassette angeordneten bzw. ausgebildeten Anschlusselementen und/oder aber auch beispielsweise an der genannten Anlagefläche. Der Pumpschlauch ist in der Schlauchkassette so angeordnet, dass er an der Anlagefläche anliegt, sodass der Pumpschlauch, wenn die Schlauchkassette auf ein Pumprad aufgesetzt ist, zwischen dem Außenumfang des Pumprades und der Anlagefläche gelegen ist. Eine solche Schlauchkassette ist vorzugsweise als austauschbarer, zum Einmalgebrauch vorgesehener Artikel ausgebildet, sodass durch Ersetzen der Schlauchkassette ein Schlauchpumpen-Aggregat sehr einfach für den nächsten Einsatz vorbereitet werden kann, ohne das fluidführende Leitungen gereinigt werden müssen. Die Anlagefläche ist vorzugsweise als bogenförmige Fläche ausgebildet, welche sich konzentrisch zu der Drehachse eines zum Eingriff in die Schlauchkassette vorgesehenen Pumprades erstreckt. Die Schlauchkassette weist dazu weiter bevorzugt Eingriffs- oder Befestigungselemente auf, welche dazu ausgebildet sind, die Schlauchkassette in definierter Position an einem Gehäuse eines Schlauchpumpen-Aggregates zu fixieren, sodass der Pumpschlauch und die Anlagefläche in eine definierte Position relativ zu dem Pumprad gebracht werden.

Der Pumpschlauch ist vorzugsweise an seinen Axialenden in der Schlauchkassette fixiert, vorzugsweise an jeweils einem Anschlusselement, welches eine Verbindung für zumindest einen Anschlussschlauch aufweist oder mit einem Anschlussschlauch verbunden ist. Die Anschlusselemente können in die Struktur der Schlauchkassette eingesetzt und in dieser fixiert sein oder auch einstückig mit der übrigen Struktur der Schlauchkassette ausgebildet sein. Zur Aufnahme des Pumpschlauches und der Anschlussschläuche sind an dem Anschlusselement vorzugsweise Pumpschlauchstutzen bzw. Anschlussstutzen ausgebildet, auf welche die Schläuche aufgesteckt sind. Bezüglich weiterer bevorzugter Ausgestaltungen wird auf die vorangehende Beschreibung des Schlauchpumpen-Aggregates verwiesen.

Nachfolgend wird die Erfindung beispielhaft anhand der beigefügten Figuren beschrieben. In diesen zeigt:
- Fig. 1: ein medizinisches Schlauchpumpen-Aggregat gemäß der Erfindung ohne Schlauchkassette,
- Fig. 2: ein medizinisches Schlauchpumpen-Aggregat gemäß Figur 1 mit aufgesetzter Schlauchkassette,
- Fig. 3: eine Frontansicht des Schlauchpumpen-Aggregates gemäß Figur 1,
- Fig. 4: eine Frontansicht des Schlauchpumpen-Aggregates mit Schlauchkassette gemäß Figur 2,
- Fig. 5: eine perspektivische Ansicht einer Rückseite der Schlauchkassette,
- Fig. 6: eine Draufsicht auf die Rückseite der Schlauchkassette gemäß Figur 5,

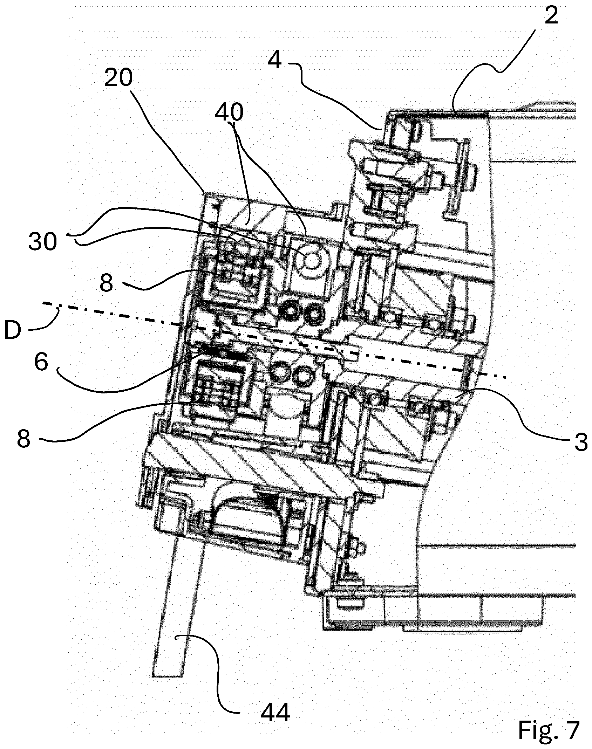

- Fig. 7: eine teilweise geschnittene Ansicht des medizinischen Schlauchpumpen-Aggregates gemäß Figur 2,

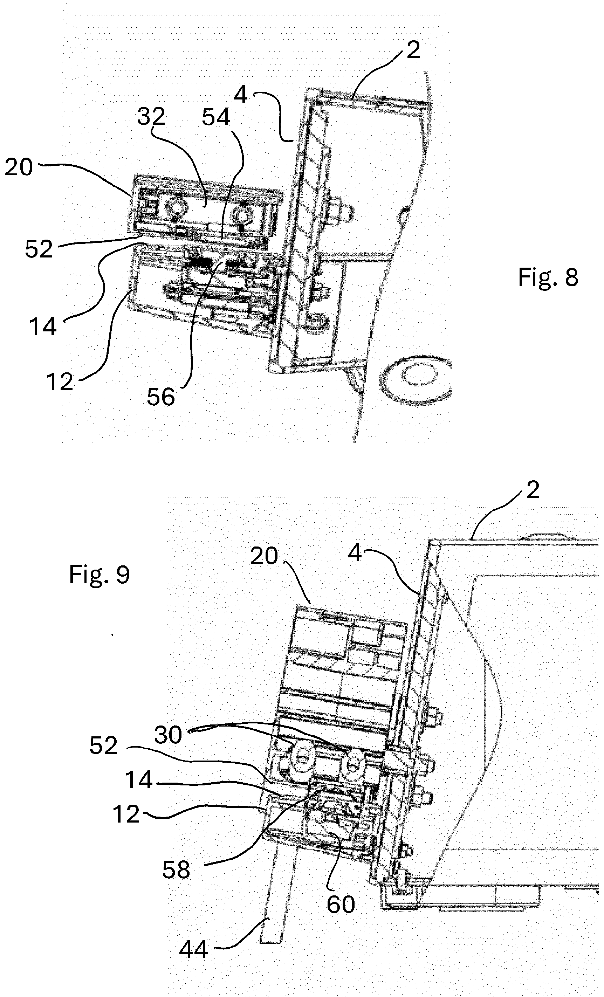

- Fig. 8: eine Schnittansicht durch einen ersten Teil der Anlagefläche und der Gegenanlagefläche sowie
- Fig. 9: einen Schnitt durch einen zweiten Bereich der Anlagefläche mit gegenüberliegender Gegenanlagefläche.

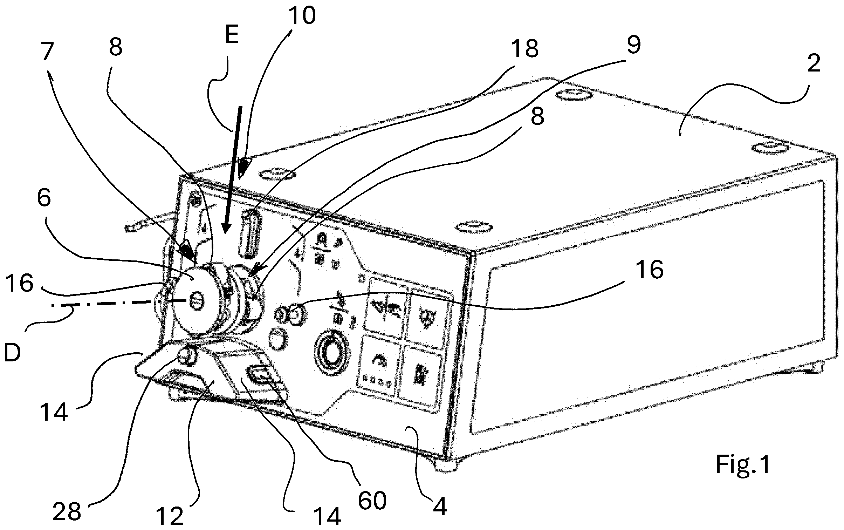

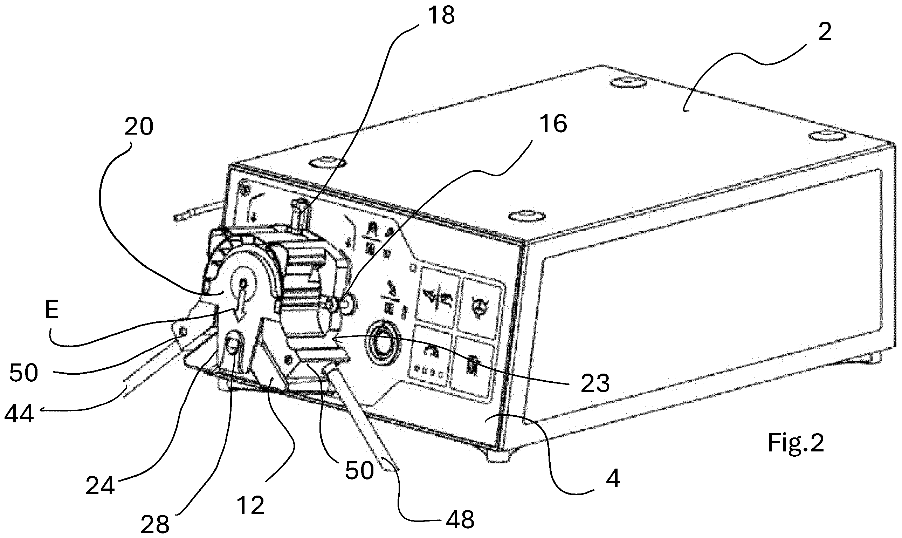

Die Figuren 1 und 2 zeigen den Gesamtaufbau eines erfindungsgemäßen medizinischen Schlauchpumpen-Aggregates. Das medizinische Schlauchpumpen-Aggregat weist ein Motorgehäuse 2 auf, in welchem zumindest ein elektrischer Antriebsmotor, eine Motorsteuerung und ggf. weitere elektronische und elektrische Bauteile zum Betrieb der medizinischen Schlauchpumpe und ggf. weiterer medizinischer Instrumente angeordnet sein können. An einer Außenwandung bzw. Außenseite, in diesem Fall der Frontseite 4 ist ein Pumprad 6 angeordnet, welches über die Welle 3 von dem elektrischen Antriebsmotor im Inneren des Motorgehäuses 2 um die Drehachse D drehend angetrieben werden kann. Das Pumprad weist in diesem Ausführungsbeispiel zwei in Richtung der Drehachse D versetzte Ebenen 7, 9 auf, in welcher jeweils zwei Verdrängerkörper in Form von Verdrängerrollen 8 angeordnet sind. Die Verdrängerrollen 8 in den beiden Ebenen 7, 9 sind um 90° versetzt zueinander angeordnet. In jeder der beiden Ebenen 7, 9 sind die zwei Verdrängerrollen 8 um 180° zueinander beabstandet.

Das Pumprad 6 ist an der Frontseite 4 im Bereich einer Kassettenaufnahme 10 angeordnet. Die Kassettenaufnahme 10 weist unterhalb des Pumprades 6 einen von der Frontseite 4 vorstehenden Anlagekörper 12 auf, welche an seiner Oberseite eine Gegenanlageflächen 14 für eine Schlauchkassette bildet. Seitlich des Pumprades 6 weist die Kassettenaufnahme darüber hinaus zwei von der Frontseite 4 vorstehende Halteelemente 16 auf. Die Halteelemente 16 sind pilzförmig ausgebildet und können mit ihren auskragenden Köpfen seitliche Abschnitte einer Schlauchkassette 20 übergreifen. Oberhalb des Pumprades 6 ist darüber hinaus ein Führungssteg 18, welcher von der Frontseite 4 vorsteht, ausgebildet. Der Führungssteg 18 erstreckt sich parallel zu einer Einsetzrichtung E, in welcher eine Schlauchkassette 20 in die Kassettenaufnahme 10 eingesetzt werden kann.

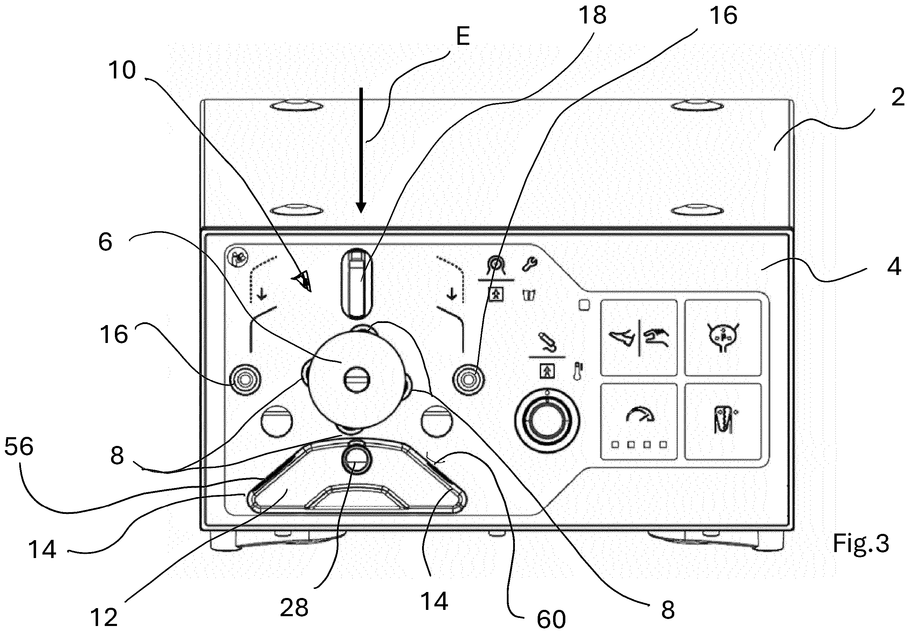

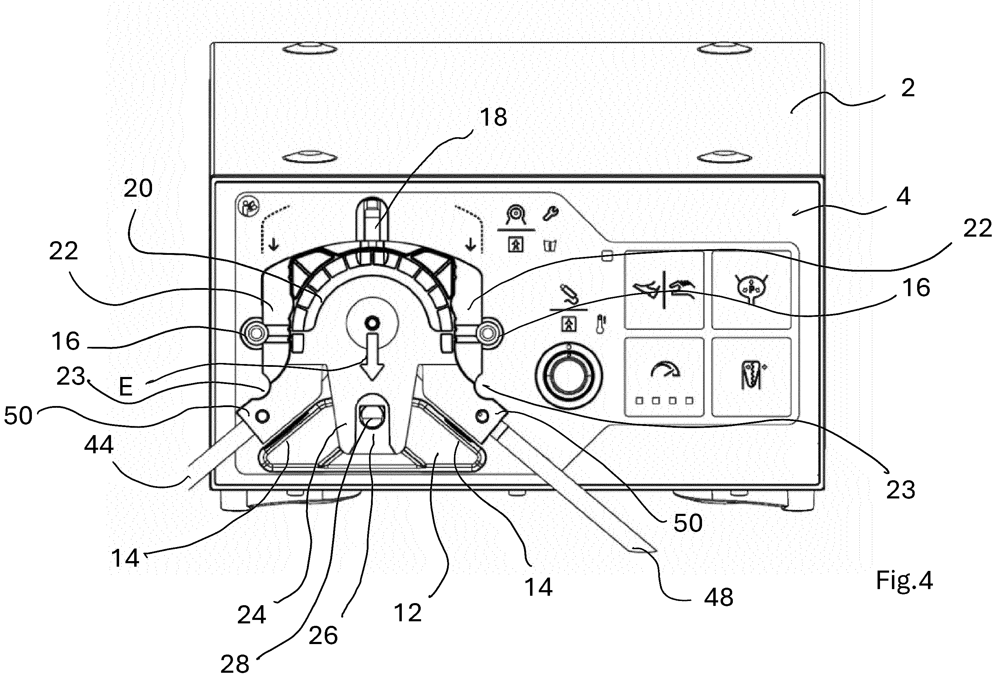

Während die Figuren 1 und 3 das Motorgehäuse 2 ohne die angesetzte Schlauchkassette 20 zeigen, ist in den Figuren 2 und 4 entsprechend das Motorgehäuse 2 mit der angesetzten Schlauchkassette 20 gezeigt. Die Schlauchkassette 20 wird zum Einsetzen in die Kassettenaufnahme zunächst parallel zur Drehachse D und dann in der Einsetzrichtung E in die Kassettenaufnahme 10 eingeschoben. Dabei wird die Schlauchkassette 20 geführt von dem Führungssteg 18 und den Halteelementen 16 in der Einsetzrichtung E parallel zur Oberfläche der Frontseite 4 über das Pumprad 6 geschoben, bis die Schlauchkassette 20 an den Gegenanlageflächen 14 an der Oberseite des Anlagekörpers 12 anliegt. Zum Halten der Schlauchkassette 20 an der Frontseite 4 weist die Schlauchkassette 20 zwei seitliche Eingriffsabschnitte 22 auf. Diese erstrecken sich quer zur Einsetzrichtung E laschen- bzw. rippenförmig nach außen und werden von den Halteelementen 16 übergriffen. Wenn die Schlauchkassette 20 beim Einsetzen zunächst quer zur Frontseite 4 bewegt wird, greifen die Halteelemente unterhalb der Eingriffsabschnitte 22 jeweils in eine Ausnehmung 23 ein. Wenn die Schlauchkassette 20 anschließend in der Einsetzrichtung E parallel zur Fronseite 4 verschoben wird, greifen die Eingriffsabschnitte 22 hinter die pilzförmigen Köpfe der Halteelemente 16. Ferner weist die Schlauchkassette 20 an ihrem in Einsetzrichtung E vorderen Ende eine Lasche 24 mit einer Öffnung 26 auf. In die Öffnung 26 greift, wenn die Schlauchkassette 20 in die Kassettenaufnahme 10 eingesetzt ist, ein federnder Rastvorsprung 28 ein, welcher an dem Anlagekörper 12 gelagert ist. Durch Druck auf den Rastvorsprung 28 kann dieser von der Öffnung 26 außer Eingriff gebracht werden und die Schlauchkassette 20 kann entgegen der Einsetzrichtung E aus der Kassettenaufnahme 10 entnommen werden.

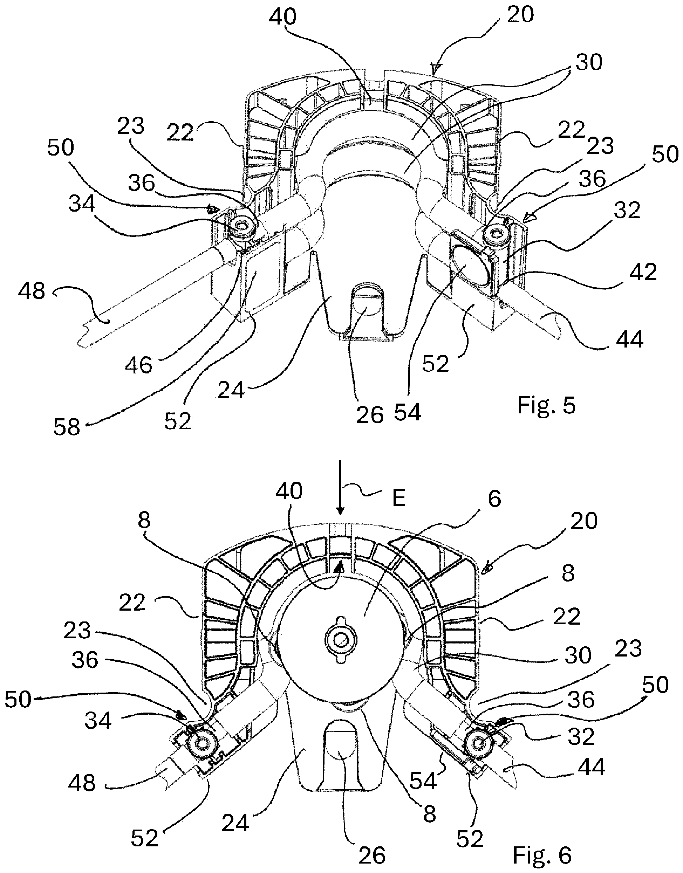

Die Figuren 5 und 6 zeigen Ansichten der Rückseite der Schlauchkassette 20, welche beim Einsetzen in die Kassettenaufnahme 10 der Frontseite 4 zugewandt ist. Die Schlauchkassette 20 ist als formstabile Struktur, vorzugsweise als Kunststoffspritzgussteil ausgebildet. Im Inneren der Schlauchkassette 20 sind zwei parallele Pumpschläuche 30 angeordnet. Die Pumpschläuche 30 sind an ihren Enden jeweils mit einem Anschlusselement 32, 34 verbunden. Die Anschlusselemente 32 und 34 sind hier als Einsätze ausgebildet, welche in die Struktur der Schlauchkassette 20 eingesetzt sind, könnten jedoch auch einstückig mit der weiteren Struktur ausgebildet sein. Jedes der Anschlusselemente 32, 34 weist zwei Pumpschlauchstutzen 36 für die Pumpschläuche 30 auf. Die Pumpschläuche 30 sind mit ihren Enden auf die Pumpschlauchstutzen 36 aufgesteckt. Im Inneren der Schlauchkassette 20 liegen die Pumpschläuche 30 an einer bogenförmigen Anlagefläche 40 an. Die bogenförmige Anlagefläche 40 erstreckt sich konzentrisch zu der Drehachse D, wenn die Schlauchkassette 20 in die Kassettenaufnahme 10 eingesetzt ist. Die Anlagefläche 40 bildet ein Widerlager. Im Pumpbetrieb drücken die Verdrängerrollen 8 die Pumpschläuche 30 gegen die Anlagefläche 40. Insofern ist der Radius der bogenförmigen Anlagefläche 40 so gewählt, dass er einen entsprechend abgestimmten Abstand zu der Drehachse D hat, wenn die Schlauchkassette 20 in die Kassettenaufnahme 10 eingesetzt ist.

Die Anschlusselemente 32, 34 sind im Wesentlichen rohrförmig ausgebildet, wobei sich die Pumpschlauchstutzen 36 jeweils quer zur Längserstreckung der Anschlusselemente 32, 34 erstrecken. Das Anschlusselement 32 weist einen Anschlussstutzen 42 auf, welcher sich bezüglich der Längsachse des Anschlusselementes 32 radial entgegengesetzt zu den Pumpschlauchstutzen 36 erstreckt. Auf den Anschlussstutzen 42 ist ein Austrittsschlauch 44 aufgesetzt. Das Anschlusselement 34 weist entsprechend einen Anschlussstutzen 46 auf, welcher sich bezüglich der Längsachse des Anschlusselementes 34 radial entgegengesetzt zu den Pumpschlauchstutzen 36 erstreckt und auf welchen ein Eingangsschlauch 48 aufgesteckt ist. Die Anschlussstutzen 42 und 46 erstrecken sich in einem spitzen Winkel zur Einsetzrichtung E, in diesem Beispiel in einem Winkel von ungefähr 45°. Dies bewirkt, dass sich der Eingangsschlauch 48 und der Austrittsschlauch 44 im Betrieb schräg nach unten erstrecken. Die Pumpschlauchstutzen 36 erstrecken sich in paralleler Richtung zu den Anschlussstutzen 42 und 46, sodass sich der sich anschließende Pumpschlauch 30 jeweils zunächst radial in Richtung auf das Pumprad 6 erstreckt und dann in tangentialer Richtung entlang der Anlagefläche 40 bogenförmig umgelenkt wird. Dies begünstigt eine Strömungsrichtung von dem Eingangsschlauch 48 in die Pumpschläuche 36 und aus den Pumpschläuchen 36 in den Austrittsschlauch 44 mit nur wenigen Richtungsänderungen, sodass insgesamt ein geringer Strömungswiderstand erreicht werden kann. In diesem Ausführungsbeispiel erstrecken sich die Pumpschlauchstutzen 36 an dem zweiten Anschlusselement 34 mit ihren Längsachsen in einem Winkel von 90° zu den Längsachsen der Pumpschlauchstutzen 36 an dem ersten Anschlusselement 32, wobei sich die Längsachsen im Wesentlichen im Mittelpunkt bzw. in der Drehachse D des Pumprades 6 schneiden.

Die Schlauchkassette 20 weist in ihrem Inneren eine im Wesentlichen U-förmige Form auf, welche durch die bogenförmige Anlagefläche 40 definiert wird. An den offenen Enden dieser U-förmigen Form erstrecken sich Schenkel 50 schräg nach außen, in denen die Anschlusselemente 32 und 34 angeordnet sind. Die Schenkel 50 erstrecken sich dabei in der Richtung der Pumpschlauchstutzen 36. Die unteren sich schräg zur Einsetzrichtung E erstreckenden und einander zugewandten Seiten der Schenkel 50 bilden Anlageflächen 52, welche an den Gegenanlageflächen 14 des Anlagekörpers 12 anliegen, wenn die Schlauchkassette 20 in die Kassettenaufnahme 10 eingesetzt ist. D.h. die Anlageflächen 52 erstrecken sich im selben Winkel zu der Einsetzrichtung E, wie die Gegenanlageflächen 14.

Das Anschlusselement 32 weist einen Innenraum auf, welcher bis an die Anlagefläche 52 grenzt und im Bereich der Anlagefläche 52 durch eine Membran 54 verschlossen ist. D.h. die Membran 54 erstreckt sich in der Anlagefläche 52 und bildet in diesem Ausführungsbeispiel zumindest näherungsweise 50% der Anlagefläche 52. Die Membran 44 ist somit vom Druck des sich im Inneren des Anschlusselementes 32 befindlichen Fluids beaufschlagt. In einem der Membran 54 zugewandten bzw. gegenüberliegenden Bereich der Gegenanlagefläche 14 ist ein Drucksensor 56 angeordnet. Wenn die Schlauchkassette 20 in die Kassettenaufnahme 10 eingesetzt ist, liegt der Drucksensor 56 an der Membran 54 an und kann auf diese Weise den Druck im Innenraum des Anschlussstutzens 32 erfassen. In diesem Ausführungsbeispiel ist dies der auslassseitige Druck, d.h. der im Austrittsschlauch 44 herrschende Druck. Die Überwachung des austrittsseitigen Drucks kann beispielsweise sinnvoll sein, um einen Überdruck zu erkennen, welcher zum Abspringen des Schlauches oder zum Platzen eines an das Schlauchpumpenaggregat angeschlossenen Auffangbehälters führen könnte. Es ist zu verstehen, dass in entsprechender Weise auch eine Membran 54 mit einem Drucksensor 56 an dem Anschlussstutzen 34 des Eingangsschlauches 48 angeordnet sein könnte.

In dem zweiten Schenkel 50, an welchem der Eingangsschlauch 48 gelegen ist, ist eine Datenspeichereinrichtung 58, beispielsweise in Form eines RFID-Chips, angeordnet bzw. eingebettet. Die Datenspeichereinrichtung 58 kann Informationen über die Art der Schlauchkassette 20 enthalten, welche von einer Steuereinrichtung in dem Motorgehäuse 2 beispielsweise dazu genutzt werden können, den Antriebsmotor in richtiger Weise einzustellen. Alternativ oder zusätzlich könnte beispielsweise auch geprüft werden, ob es sich um eine zugelassene Schlauchkassette 20 für das jeweilige Schlauchpumpen-Aggregat handelt. Zum Lesen und/oder Schreiben der Datenspeichereinrichtung 58 ist eine Leseeinrichtung 60 bzw. Schreib-/Leseeinrichtung 60 in dem Anlagekörper 12 im Bereich der Gegenanlagefläche 14, welche der Datenspeichereinrichtung 58 gegenüberliegt, angeordnet. Die Leseeinrichtung 60 kann beispielsweise eine Antenneneinrichtung sein, welche mit einer geeignet ausgebildeten Steuerelektronik verbunden ist und zum Lesen und/oder Schreiben der Datenspeichereinrichtung 58 ausgestaltet ist. So ist in einem der Schenkel 50 die Membran 54 zur Druckmessung angeordnet, während in dem anderen Schenkel 50 die Datenspeichereinrichtung 58 angeordnet ist. Entsprechend liegt in einer der Gegenanlageflächen 14, derjenigen, die der Membran 54 zugewandt ist, der Drucksensor 56, während in der anderen Gegenanlagefläche 14 die Leseeinrichtung 60 gelegen ist.

### Bezugszeichenliste

- 2: Motorgehäuse
- 3: Welle
- 4: Frontseite
- 6: Pumprad
- 7, 9: Ebene des Pumprades
- 8: Verdrängerrollen
- 10: Kassettenaufnahme
- 12: Anlagekörper
- 14: Gegenanlagefläche
- 16: Halteelemente
- 18: Führungssteg
- 20: Schlauchkassette
- 22: Eingriffsabschnitte
- 23: Ausnehmungen
- 24: Lasche
- 26: Öffnung
- 28: Rastvorsprung
- 30: Pumpschläuche
- 32,34: Anschlusselemente
- 36: Pumpschlauchstutzen
- 40: Anlagefläche
- 42: Anschlussstutzen
- 44: Austrittsschlauch
- 46: Anschlussstutzen
- 48: Eingangsschlauch
- 50: Schenkel
- 52: Anlageflächen
- 54: Membran
- 56: Drucksensor
- 58: Datenspeichereinrichtung
- 60: Leseeinrichtung
- D: Drehachse
- E: Einsetzrichtung

## Patentansprüche

1. Medizinisches Schlauchpumpen-Aggregat mit einem Pumprad (6) und zumindest einem das Pumprad (6) in einem Umfangsbereich umschließenden Pumpschlauch (30), wobei der Pumpschlauch (30) in seinem das Pumprad (6) umschließenden Abschnitt an seiner dem Pumprad (6) abgewandten Außenseite an einem Widerlager (40) anliegt.

2. Medizinisches Schlauchpumpen-Aggregat nach Anspruch 1, bei welcher das Widerlager von einer Anlagefläche (40) und vorzugsweise von einer bogenförmigen, sich konzentrisch zur Drehachse (D) des Pumprades erstreckenden Anlagefläche (40) gebildet wird.

3. Medizinisches Schlauchpumpen-Aggregat nach Anspruch 1 oder 2, bei welchem der zumindest eine Pumpschlauch (30) in einer Schlauchkassette (20) fixiert ist, in welcher auch das Widerlager (40) ausgebildet ist.

4. Medizinisches Schlauchpumpen-Aggregat nach einem der vorangehenden Ansprüche, bei welchem das Pumprad (6) an einem Motorgehäuse (2) angeordnet ist und die Schlauchkassette (20) lösbar mit dem Motorgehäuse (2) verbindbar ist, wobei das Pumprad (6) in die Schlauchkassette (20) eingreift und mit dem zumindest einen Pumpschlauch (30) in Kontakt tritt.

5. Medizinisches Schlauchpumpen-Aggregat nach einem der vorangehenden Ansprüche, bei welchem der zumindest eine Pumpschlauch (30) mit seinen axialen Enden in der Schlauchkassette (20) fixiert ist, vorzugsweise jeweils an einem Anschlusselement (32, 34) fixiert ist, welches mit zumindest einem Anschlussschlauch (44, 48) verbunden ist oder eine Verbindung (42, 46) fürzumindest einen Anschlussschlauch (44, 48) aufweist.

6. Medizinisches Schlauchpumpen-Aggregat nach einem der vorangehenden Ansprüche, bei welchem die Schlauchkassette (20) eine offene Seitenfläche aufweist, durch welche das Pumprad (6) in das Innere der Schlauchkassette (20) eingreift.

7. Medizinisches Schlauchpumpen-Aggregat nach einem der vorangehenden Ansprüche, bei welche eine Stirnseite der Schlauchkassette (20) derart offen ausgebildet ist, dass die Schlauchkassette (20) mit der offenen Stirnseite voran über das Pumprad (6) aufschiebbar ist, wobei vorzugsweise eine das Widerlager (40) bildende Innenwandung der offenen Stirnseite zugewandt ist.

8. Medizinisches Schlauchpumpen-Aggregat nach einem der vorangehenden Ansprüche, bei welcher das Pumprad (6) zumindest einen Verdrängerkörper (8) aufweist, welcher bei Drehung des Pumprades (6) auf den zumindest einen Pumpschlauch (30) einwirkt, wobei der Verdrängerkörper (8) vorzugsweise eine in dem Pumprad (6) drehbar gelagerte Verdrängerrolle (8) ist, die derart angeordnet ist, dass sie bei Drehung des Pumprades (6) auf dem Pumpschlauch (30) abrollt.

9. Medizinisches Schlauchpumpen-Aggregat nach einem der vorangehenden Ansprüche, bei welchem der zumindest eine Verdrängerkörper, vorzugsweise die zumindest eine Verdrängerrolle (8) in dem Pumprad (6) in radialer Richtung federnd gelagert ist.

10. Medizinisches Schlauchpumpen-Aggregat nach einem der vorangehenden Ansprüche, bei welchem zumindest zwei zueinander parallel verlaufende Pumpschläuche (30) das Pumprad (6) umschlie-βen und bei welchem an dem Pumprad (6) vorzugsweise zumindest ein erster Verdrängerkörper (8), welcher auf einen ersten der Pumpschläuche (30) wirkt, und zumindest ein zweiter Verdrängerkörper (8), welcher auf einen zweiten der Pumpschläuche (30) wirkt, angeordnet sind.

11. Medizinisches Schlauchpumpen-Aggregat nach einem der vorangehenden Ansprüche, bei welchem die Schlauchkassette (20) mit dem zumindest einen Pumpschlauch (30) als austauschbarer, zum Einmalgebrauch vorgesehener Artikel ausgebildet ist.

12. Schlauchkassette (20) für ein medizinisches Schlauchpumpen-Aggregat, vorzugsweise für ein medizinisches Schlauchpumpen-Aggregat nach einem der Ansprüche 1 bis 11, welche einen Aufnahmeraum für ein Pumprad (6) aufweist, welcher an einer Seite von einer Anlagefläche (40) begrenzt ist, an welcher ein Pumpschlauch (30) derart anliegt, dass der Pumpschlauch (30) bei eingesetztem Pumprad (6) zwischen dem Außenumfang des Pumprades und der Anlagefläche (40) gelegen ist.

13. Schlauchkassette (20) nach Anspruch 12, bei welcher der zumindest einen Pumpschlauch (30) mit seinen Axialenden in der Schlauchkassette (20) fixiert ist, vorzugsweise an jeweils einem Anschlusselement (32, 34) fixiert ist, welches eine Verbindung für zumindest einen Anschlussschlauch aufweist.
